# EUROPEAN PATENT SPECIFICATION

(11) **EP 3 154 620 B1**
(45) Date of publication and mention of the grant of the patent: **09.09.2020**
(21) Application number: 15806028.5
(22) Date of filing: 12.06.2015
(51) Int. Cl.: A61B 8/12, A61B 5/00, A61B 8/08, A61B 8/14, A61M 25/10

(54) **IMAGE GUIDED THERAPEUTIC CATHETER WITH DRUG ELUTING BALLOON**
BILDGEFÜHRTER THERAPEUTISCHER KATHETER MIT WIRKSTOFFFREISETZENDEM BALLON
CATHÉTER THÉRAPEUTIQUE GUIDÉ PAR IMAGE AYANT UN BALLONNET D'ÉLUTION DE MÉDICAMENT

(30) Priority: 12.06.2014 US 201462011276 P
(43) Date of publication of application: 19.04.2017
(73) Proprietor: Koninklijke Philips N.V., 5656 AG Eindhoven (NL)
(72) Inventor: STIGALL, Jeremy, San Diego, CA 92130 (US)
(74) Representative: Philips Intellectual Property & Standards
(86) International application number: PCT/US2015/035609
(87) International publication number: WO 2015/192041

(56) References cited:
- WO-A1-2014/031736
- WO-A2-2012/092444
- US-A- 5 102 402
- US-A1- 2004 092 830
- US-A1- 2007 073 364
- US-A1- 2009 076 448
- US-A1- 2009 187 108
- US-A1- 2011 160 658
- US-A1- 2012 310 210
- US-A1- 2014 163 358
- US-A1- 2014 276 028

## Description

### TECHNICAL FIELD

Embodiments of the present disclosure relate generally to the field of medical devices and, more particularly, to integrated therapeutic imaging catheters utilizing drug eluting balloons.

### BACKGROUND

Intravascular imaging systems are widely used in interventional cardiology as a diagnostic tool for a diseased vessel, such as an artery, within the human body. Various sensors may be placed on a catheter and positioned in the body. One type of imaging system is an intravascular ultrasound ("IVUS") system. In one example, a phased array IVUS device includes a number of transducers that are passed into a vessel and guided to an area to be imaged. The transducers emit ultrasonic waves in order to create an image of the vessel of interest. The ultrasonic waves are partially reflected by discontinuities arising from tissue structures (such as the various layers of the vessel wall), red blood cells, and other features of interest. Echoes from the reflected waves are received by the transducer and passed along to an IVUS imaging system. The imaging system processes the received ultrasound echoes to produce a cross-sectional image of the vessel where the device is placed. The imaging data is thereafter utilized to treat a variety of vessel abnormalities.

Drug eluting coatings on balloon catheters ("DEB") are utilized to treat plaque in blood vessels. To be effective, the DEB coatings need to be positioned in correct location in the vessel and positioned in apposition with the stenotic lesion within the vessel. While DEB coatings have been effective, there remains a need for better placement devices and techniques.

WO 2012/092 444 A2 describes methods and devices that, in various embodiments, diagnose and treat multiple sclerosis, deep vein thrombosis, and/or pulmonary embolism or associated symptoms. In some instances, stenosis or other flow limiting structures or lesions in the patient's affected veins are identified. Further, in some instances the nature of such lesions and whether there is a significant disruption of blood pressure, or both, is ascertained. In some embodiments, methods and devices for applying one or more therapies to the blockages in the patient's internal jugular veins or azygous veins are provided.
US 2009/0187108 A1 describes a catheter which is provided for placement within a body lumen, the catheter including a flexible conduit that is elongated along a longitudinal axis, the flexible conduit having a proximal end and a distal end. The catheter further includes at least one delivery waveguide and at least one collection waveguide positioned along the flexible conduit, the at least one delivery waveguide and the at least one collection waveguide constructed and arranged to transmit radiation at a wavelength in a range of about 250 to 2500 nanometers. The catheter further includes a flexible, expandable first surface encircling surrounding a segment of the conduit.
US 2007/0073364 A1 describes a catheter device and a method for in vivo activation of a photosensitizing drug in a vessel, endovascular tissue, and/or intraluminal tissue, a catheter carrying both an optical coherence tomography (OCT) lens, from which OCT imaging light is emitted, and a photodynamic therapy (PDT) lens from which photosensitizing drug-activating light is emitted, is inserted into a vessel containing a lesion to be treated. A photosensitizing drug is caused to be placed in the vessel as well, such as in the form of a coating on a stent or a coating on an exterior of a balloon carried by the catheter. Light is emitted from the PDT lens to activate the photosensitizing drug while light is simultaneously emitted from the OCT lens to obtain an OCT image to monitor the drug activation.

### SUMMARY

The invention is defined by independent claim 1. The present disclosure provides devices and systems for imaging and treating diseased vessels using a drug eluting balloon ("DEB") assembly. In one illustrative embodiment, the present disclosure describes an integrated therapeutic and imaging catheter having an imaging device and DEB assembly. The DEB assembly is a balloon assembly having its outer surface coated with a drug eluting coating. In a generalized method, the catheter is inserted into a patient's vessel, whereby the vessel walls are imaged by the imaging device. After identification of a treatment area (e.g., a lesion, stenosis or nerve plexus) within or along the vessel, the DEB assembly is inflated to thereby apply the drug eluting coating to the treatment area. After application, the treatment area and the drug eluting coating may be re- imaged in order to determine if the treatment area was sufficiently coated. If the treatment area is found to be insufficiently coated, the DEB assembly may be re-inflated to force the drug eluting coating into complete contact with the treatment area.

### BRIEF DESCRIPTION OF THE DRAWINGS

Figs. 1 and 2 are diagrams showing illustrative DEB imaging catheters, according to certain illustrative embodiments of the present disclosure;
Figs. 3 and 4 are diagrams showing illustrative cross-sections taken along line 2-2 of Figs. 1 and 4, respectively, of a proximal junction of a DEB imaging catheter, according to certain illustrative embodiments of the present disclosure;
Figs. 5 and 6 are diagrams showing illustrative cross-sections of a DEB assembly taken along line 3-3 of Figs. 1 and IB, respectively, according to certain illustrative embodiments of the present disclosure;

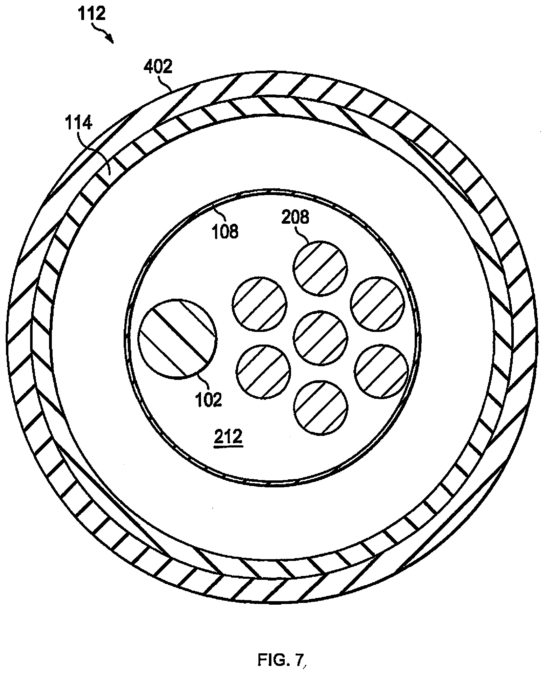

Figs. 7 and 8 are diagrams showing illustrative cross-sections of a distal junction of a DEB catheter taken along line 4-4 of Figs. 1 and IB, respectively, according to certain illustrative embodiments of the present disclosure;
Figs. 9-14 are diagrams showing illustrative insertions of a DEB imaging catheter into a vessel of a patient according to certain illustrative methods of the present disclosure; and
Figs. 15 and 16 are diagrams showing illustrative cross-sections taken along line 6-6 of Figs. 12 and 14, respectively, of the junction of a drug eluting coating and lesion, according to certain illustrative embodiments of the present disclosure.

### DETAILED DESCRIPTION OF ILLUSTRATIVE EMBODIMENTS

For the purposes of promoting an understanding of the principles of the present disclosure, reference will now be made to the embodiments illustrated in the drawings, and specific language will be used to describe the same. It will nevertheless be understood that no limitation of the scope of the disclosure is intended. Any alterations and further modifications in the described devices, instruments, methods, and any further application of the principles of the disclosure as described herein are contemplated as would normally occur to one ordinarily skilled in the art to which the disclosure relates. In particular, it is fully contemplated that the features, components, and/or steps described with respect to one embodiment may be combined with the features, components, and/or steps described with respect to other embodiments of the present disclosure.

As described herein, illustrative embodiments of the present disclosure are directed to integrated imaging catheters that include a DEB assembly having a drug eluting coating placed thereon to thereby accurately access, assess, and treat diseased vessels within a patient. The imaging devices may be, for example, a transducer or optical device operable to perform sensing modalities such as IVUS, optical coherence tomography ("OCT"), photo acoustic inspection and spectroscopy. In some embodiments, the imaging device elements may be oriented generally perpendicular to the axis of the device for side looking imaging, while other embodiments may employ axially oriented imaging sensors that provide forward looking imaging ahead of the balloon assembly. Moreover, the embodiments disclosed herein provide a drug eluting coating on the DEB assembly which, upon inflation of the DEB assembly, applies the drug eluting coating to a treatment area within the vessel.

In an illustrative generalized method of the present disclosure, the DEB catheter is inserted into the patient's vessel and a treatment area within the lumen of the vessel is imaged by the imaging device. Once the treatment area has been identified via the imaging assembly, the DEB assembly is positioned adjacent the treatment area and inflated at a first pressure. As the balloon is inflated, the drug eluting coating is applied to the treatment area. In certain methods, after initial application of the drug eluting coating, the imaging device re-images the treatment area to assess the apposition *(i.e.,* relative position) of the drug eluting coating and lesion. If the apposition is incomplete *(i.e.,* there are discontinuities between the drug eluting coating and treatment area), the balloon assembly is re-inflated at a second higher pressure to eliminate the discontinuities, thereby resulting in a complete apposition of the drug eluting coating and treatment area.

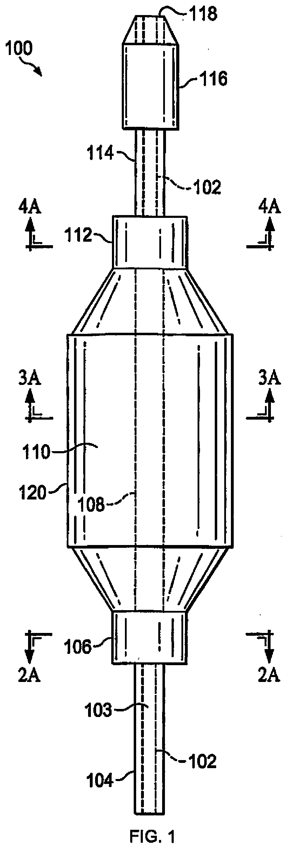

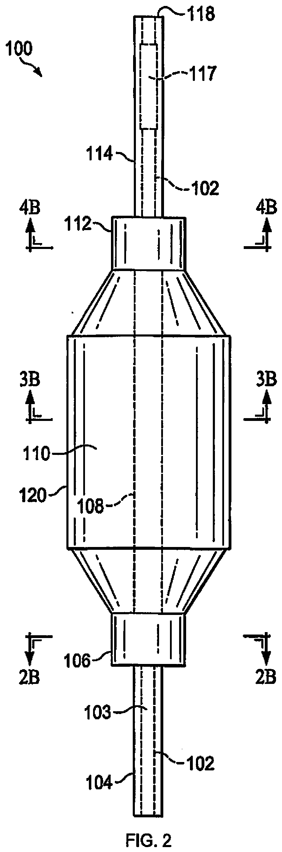

Figs. 1 and 2 are diagrams showing an illustrative DEB catheter 100 according to certain embodiments of the present disclosure. Fig. 1 illustrates a DEB catheter 100 having an electronically actuated sensor 116 *(i.e.,* imaging device), while Fig. 2 illustrates a DEB catheter having a sensor 117 *(i.e.,* imaging device) that is rotated by a drive shaft. As will be understood by those ordinarily skilled in the art having the benefit of this disclosure, sensor 116 is fixed and cannot move relative to other elements of DEB catheter 100, while sensor 117 is rotatable and sized such that it can be moved longitudinally inside mid-shaft 114 relative to other elements of DEB catheter 100 as will be described below. The components of the systems have many common elements which may be referred to by the same reference numbers throughout the disclosure. According to certain illustrative embodiments, DEB catheter 100 includes a DEB assembly 110 *(i.e.,* balloon assembly) with an inner sleeve 108 and an outer sleeve upon which a drug eluting coating 120 is positioned. The DEB assembly 110 is joined to a proximal shaft 104 through a proximal junction 106. Additionally, DEB assembly 110 is joined to a mid-shaft 114 through a distal junction 112. In the illustrated embodiment, mid-shaft 114 extends between DEB assembly 110 and sensing device 116. An inner member 102 defining a guide wire lumen 103 runs from the tip 118 of the catheter, through the interior of proximal shaft 104, DEB assembly 110, and mid-shaft 114, to at least the proximal end of DEB assembly 110.

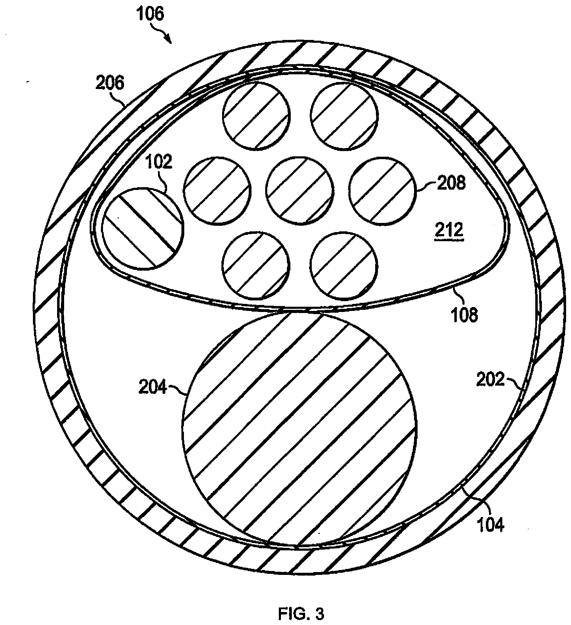

Proximal shaft 104 connects DEB assembly 110 to a pressurized fluid system while a connection medium 208 (Figs. 3-4), such as electrical conductors or optical fibers, extending within proximal shaft 104 connect sensing device 116 to a processing system (not shown) at the proximal end of DEB catheter 100. In certain embodiments, sensing device 116 is an ultrasound transducer array having a maximum outer diameter of 3.14 and connection medium 208 is a microcable having a braided exterior with 7 individual insulated electrical conductors. In other embodiments, connection medium 208 comprises fiberoptics. In yet other embodiments, connection medium 208 extends through the entire length of DEB assembly 110 and joins sensing device 116.

The processing systems typically remain outside of the patient; however, the present disclosure is not limited to such embodiments. As will be understood by those ordinarily skilled in the art having the benefit of this disclosure, the processing system uses the data received from sensing device 116. When sensing device 116 is part of an imaging system, the data can be used to create an image. The image can be displayed to a medical professional in real time as the DEB catheter moves through the patient's artery. This allows the medical professional to find various occlusions or other irregularities *(i.e.,* treatment areas) which may exist throughout the patient's vessel. In certain embodiments, sensing device 116 could be a pressure or flow sensor, and the processing system could determine fractional flow reserve values based on the sensed data.

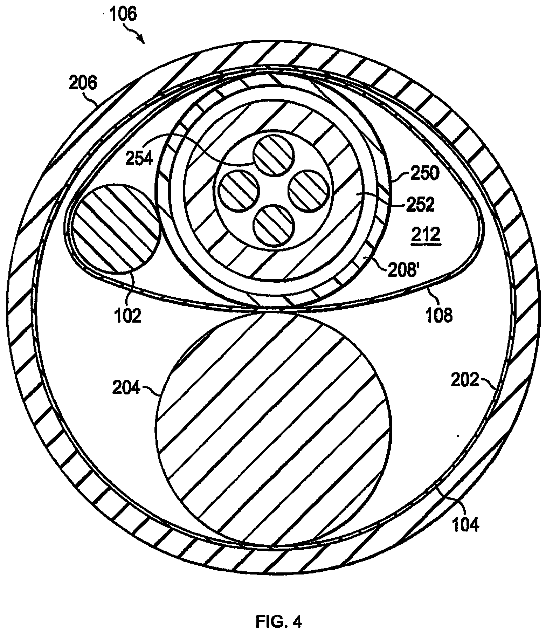

Still referring to Figs. 3 and 4, proximal shaft 104 may be made of a plastic, polymer, metal, or other flexible material. In one example, proximal shaft 104 may include a metal proximal portion joined to a distal polymer tube with a metal wire embedded in the polymer tubing adjacent the coupling to transition the stiffness of the tubing from the stiffer metal to the more flexible polymer tubing. Proximal shaft 104 is designed to be flexible so that it may effectively traverse a patient's blood vessel without damaging the vessel. Proximal shaft 104 may be a dual lumen shaft in certain embodiments. The dual lumen proximal shaft 104 may be an axial dual lumen shaft with an inner lumen and an outer lumen.

In certain illustrative embodiments, proximal shaft 104 may have a diameter within the range of 2 to 4 French (i.e., 0.67 to 1.33 mm). The length of proximal shaft 104 is long enough to allow DEB assembly 110 and sensing device 116 to reach a sufficiently deep region of a patient's vessel. For example, proximal shaft 104 may have a length of approximately 150 cm. In a collapsed condition, the maximum outer diameter of DEB assembly 110 is approximately 0.040 inches.

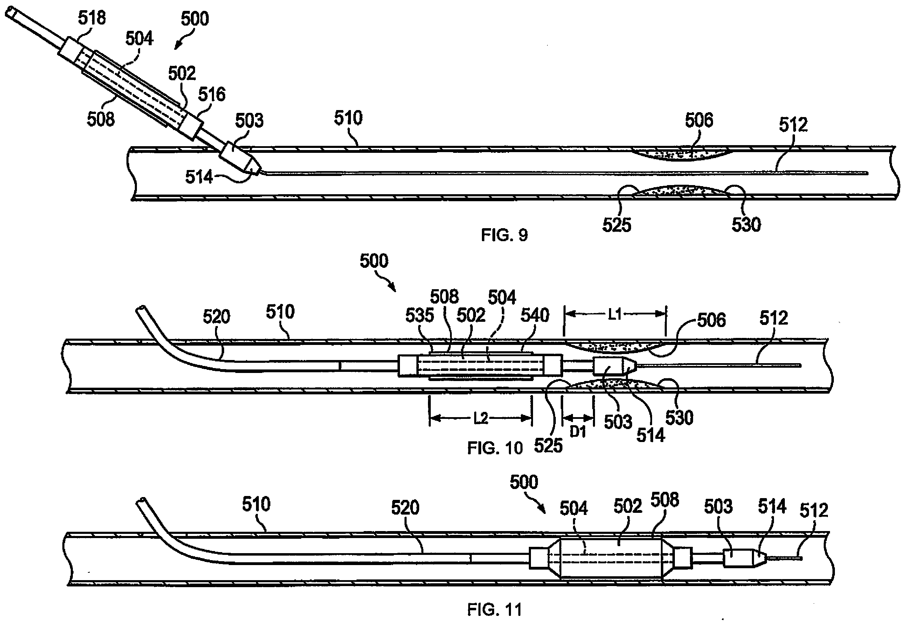

Inner member 102 defines a guidewire lumen 103 that is sized to receive a guide-wire (shown in Fig. 9). In one embodiment, guidewire lumen 103 has a diameter of 0.017 inches such that it can receive a 0.014 inch diameter guidewire. Typically, a guide-wire is first inserted into a patient's vessel. The catheter is then placed over the guide-wire such that inner member 102 encompasses the guide-wire. In some examples, inner member 102 may extend the entire length of the DEB catheter 100, from tip 118 to the proximal end of proximal shaft 104. Such a catheter is referred to as an over-the-wire catheter. In some examples, inner member 102 may extend along a short distance and then exit out of DEB catheter 100 at an exit port near the proximal end of DEB assembly 110. Such a catheter is referred to as a rapid exchange catheter.

The length of inner member 102 is long enough to extend from the point at which the catheter starts on the guide-wire (typically, the tip) to the point at which the guide-wire exits the catheter. Thus, the length may be relatively short in the case of a rapid exchange catheter and relatively long in the case of an over-the-wire catheter. Nevertheless, in this example, mid-shaft 114 is connected between the distal end of DEB assembly 110 and sensing device 116. Midshaft 114 may be made of a polymer, plastic, or other flexible material. Mid-shaft 114 is flexible so that it may effectively traverse a patient's artery without damaging the artery. Inner member 102 runs through the interior of the mid-shaft 114. Additionally, as described in more detail below, the connection medium runs from sensing device 116 towards DEB assembly 110 through mid-shaft 114.

Fig. 3 is a diagram showing an illustrative cross-section of proximal junction 106 of the DEB catheter 100 according to one illustrative embodiment of the present disclosure. Proximal junction 106 connects the proximal end of DEB assembly 110 to the proximal shaft (e.g., 104, Fig. 1). According to certain illustrative examples, proximal shaft 104 is a dual lumen shaft that includes an inner lumen 204 and an outer lumen 202. Proximal junction 106 also includes the inner member 102, inner balloon sleeve 108, and a space through which connection media 208 run. Proximal junction 106 further includes a balloon proximal leg 206. In one aspect, the balloon proximal leg 206 is an extension of the material forming the balloon outer sleeve upon which drug eluting coating 120 is positioned.

Fig. 4 illustrates a cross-sectional view of the embodiment shown in Fig. IB. The embodiment of Fig. 4 includes an alternative connection media 208'; formed as a rotary drive cable assembly. The cable includes an outer sheath 250 surrounding an inner drive cable 252 and a series of electrical conductors or optical fibers 254.

The outer lumen 202 of proximal shaft 104 provides an external structure for the proximal shaft 104. Inner lumen 204 is smaller in diameter than outer lumen 202 and runs axially within outer lumen 202. The size of inner lumen 204 is such that there is sufficient room within the outer lumen for inner member 102, inner balloon sleeve 108, and connection media 208.

In this illustrative embodiment, inner lumen 204 can be used to pump inflation fluid into DEB assembly 110. Thus, the end of the inner lumen 204 within proximal junction 106 serves as an inflation port where the inflation fluid exits inner lumen 204 into DEB assembly 110, as will be understood by those ordinarily skilled in the art having the benefit of this disclosure. The inflation fluid exits into the space between balloon inner sleeve 108 and the balloon outer sleeve, thus inflating the balloon.

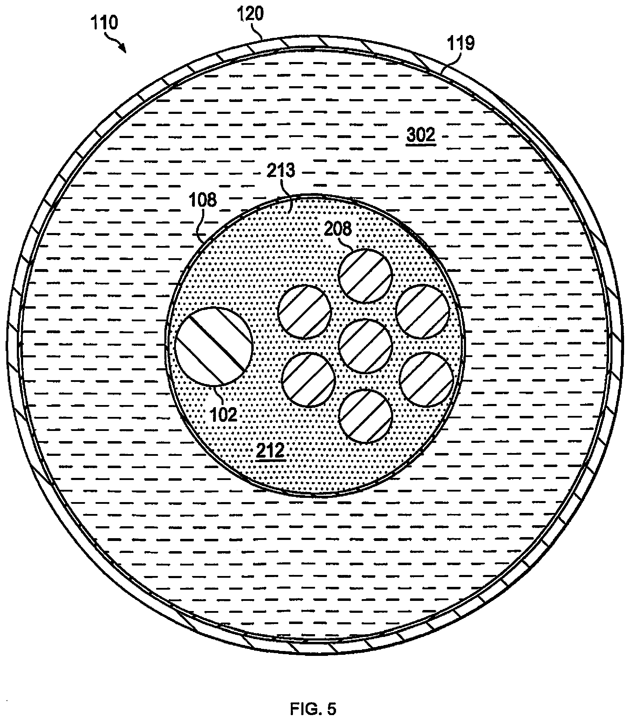

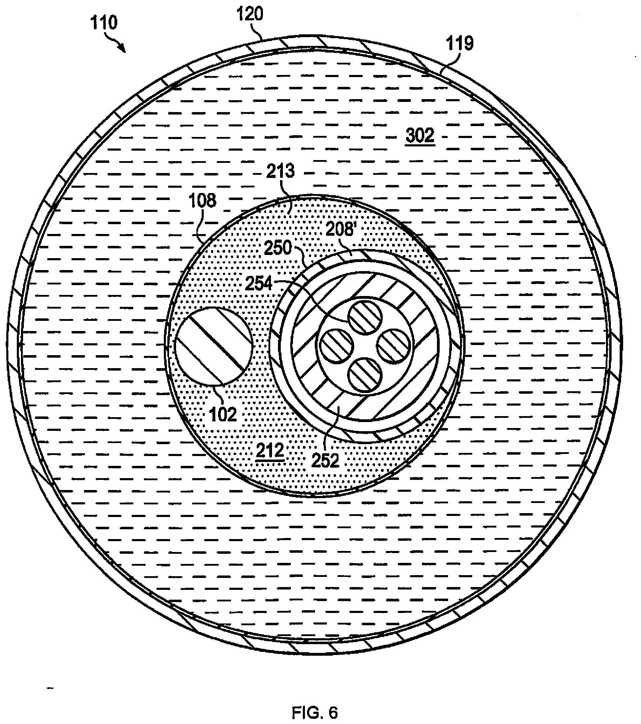

The balloon inner sleeve 108 acts as a barrier between the inflation fluid and any structures that run through the internal portion of DEB catheter 100, particularly, the connection media 208 and inner member 102. Balloon inner sleeve 108 is bonded to the interior of the outer lumen 202 of proximal shaft 104. Additionally, balloon inner sleeve 108 encompasses inner member 102. As shown more fully in Figs. 5 and 6, balloon inner sleeve 108 is sized such that there is a sufficient space 212 between sleeve 108 and inner member 102 so as to allow any connection media 208 or 208' to fit therein. This space 212 allows connection media 208 or drive cable 208' to float freely without damaging the integrity of DEB assembly 110. However, bonding material 213 (Figs. 5-6) fills the space in proximal connection 106 and distal connection 112 to define the fluid tight region 212 within inner sleeve 108 inside DEB assembly 110.

In one example, inner sleeve 108 is formed of a multi-layer structure suitable for high pressure operation greater than 20 atmospheres ("ATM"). In some embodiments, inner sleeve 108 is configured to be suitable for operating pressures extending through, by way of example only, a range of 15 to 25 ATM. In one embodiment, this range may comprise 17 to 22 ATM. In another embodiment, this range may comprise 19 to 21 ATM. Other ranges are contemplated. The material properties and construction of inner sleeve 108 allow it to deform under high pressure without significant elongation along the longitudinal axis of DEB assembly 110, even under the application of high pressures. In some embodiments, the materials forming inner sleeve 108 permit very little, if any, axial compression and extension, even under the application of high pressures.

In one embodiment, inner sleeve 108 is formed by an inner layer of polyethylene ("PE") bonded to an outer layer of maleated polyethylene. The outer layer of maleated PE is more suitable for heat treated bonding to other components of the system, such as proximal shaft 104 and mid-shaft 114, that can be formed of PBAX™. It will be understood by those ordinarily skilled persons described herein that proximal shaft 104, mid-shaft 114, and inner shaft 102 are formed such that they do not deform under high operating pressures, while inner sleeve 108 is designed to intentionally elastically deform inwardly under the high operating pressures of the balloon system. Inner sleeve 108 is shaped and configured to collapse around connection media 208 or drive cable 208' without damaging or otherwise interfering with the operation of the connection media or drive cable running through inner sleeve 106. Inner sleeve 108 then elastically returns to its original shape when the high pressure condition is removed. Return of inner sleeve 108 to its original shape may also be aided by the compressed gas within space 212.

In certain illustrative embodiments, various types of connection media may run through space 212 between inner member 102 and inner sleeve 108. For example, in the case that sensing devices 116, 117 produce electrical signals to be processed by external systems, then connection media 208, 208' may include conductive wires to carry those electrical signals. Alternatively, the connection media may include fiber optic cables to propagate those signals in the form of light. The number of wires or cables depends on the type of sensing device and the manner in which data is transferred from the sensing device to the external processing systems. Conductive wires may also be used to provide electrical power to the sensing device.

In the case that the sensing device is rotational (i.e., sensing device 117), connection media 208' may include a driveshaft lumen. In one aspect, the driveshaft lumen may include a plastic sheath, such as sheath 250, filled with a liquid lubricant. The lubricant allows the driveshaft running through the plastic sheath to spin with a minimal amount of friction against the interior of the plastic sheath.

Balloon proximal leg 206 is part of the balloon outer sleeve upon which drug eluting coating 120 is positioned (Fig. 1). Balloon proximal leg 206 is designed to fit securely around the exterior of the proximal shaft 104, as shown in Figs. 3-4. Balloon proximal leg 206 may be bonded to the exterior of proximal shaft 104 through a variety of bonding methods. These bonding methods include, but are not limited to, thermal bonding and laser bonding.

Fig. 3 A is a diagram showing an illustrative cross-section of the DEB assembly 110 taken along line 3-3 of Fig. 1. According to certain illustrative examples, the cross-section includes a balloon outer sleeve 119, balloon inner sleeve 108, connection media 208, and inner member 102. A drug eluting coating 120 is positioned on balloon outer sleeve 119 using, for example, a primer, main coat and top coat. Drug eluting coating 120 may be, for example, paclitaxel and/or Everolimus, in certain embodiments. The drug release rate may be comparable to any release rate on any clinically relevant drug eluting balloon, as will be understood by those ordinarily skilled in the art having the benefit of this disclosure. In certain embodiments, the drug utilized will biodegrade in the body and decompose within a certain period of time, thereby eliminating the need for stenting. The size of the balloon of DEB assembly 110 may range from lmm-80mm in diameter and 5mm-300mm in length, in certain embodiments. Ultimately, there are a variety of drug eluting balloons which may be utilized with embodiments of the present disclosure, as will be understood by those ordinarily skilled in the art having the benefit of this disclosure.

The diameter of DEB assembly 110 depends on the amount of inflation fluid 302 pumped into DEB assembly 110 through the proximal junction. For non-distensible balloon materials, the balloon diameter is fixed to a specific diameter. In one embodiment, the non-compliant balloon has a working length of approximately 15 mm and is available in expanded diameters ranging from 2.0 to 4.0 mm in 0.5 mm increments. In another embodiment, the outer diameter of the balloon assembly in the collapsed state is approximately 0.040 inches.

Proximal shaft 104 at the proximal end of DEB assembly 110 and mid-shaft 114 at the distal end of DEB assembly 110 are independent shafts. According to certain illustrative embodiments, there is not a continuous shaft extending through the interior of DEB assembly 110. Rather, the interior of DEB assembly 110 includes only connection media 208 and inner member 102 (through which the guidewire may run). This provides additional flexibility within DEB assembly 110. Moreover, this allows connection media 208 to float freely within space 212 between balloon inner sleeve 108 and inner member 102. In the illustrated example, the ends of balloon inner sleeve 108 are sealed to the respective proximal and distal catheter components forming fluid tight chamber 212 surrounding microcable 208 and inner member 102. In some cases, space 212 may be filled with air or other gases, while in some cases space 212 may be filled with a liquid.

As mentioned above, an inflation fluid is used to inflate DEB assembly 110 when it is appropriately aligned in order to perform various medical tasks such as applying drug eluting coating 120 to a treatment area such as, for example, a lesion, stenosis or nerve plexus. Thus, the diameter of the balloon outer sleeve 119 and drug eluting coating 120 changes based on the inflation status of DEB assembly 110. In those embodiments in which the balloon is non-compliant, the diameter only extends to a certain point. The non-compliant nature of the balloon prevents too much expansion within a patient's artery. Balloon inner sleeve 108 is designed with integrity such that balloon inner sleeve 108 will not place too great of a pressure on connection media 208 when DEB assembly 110 is inflated.

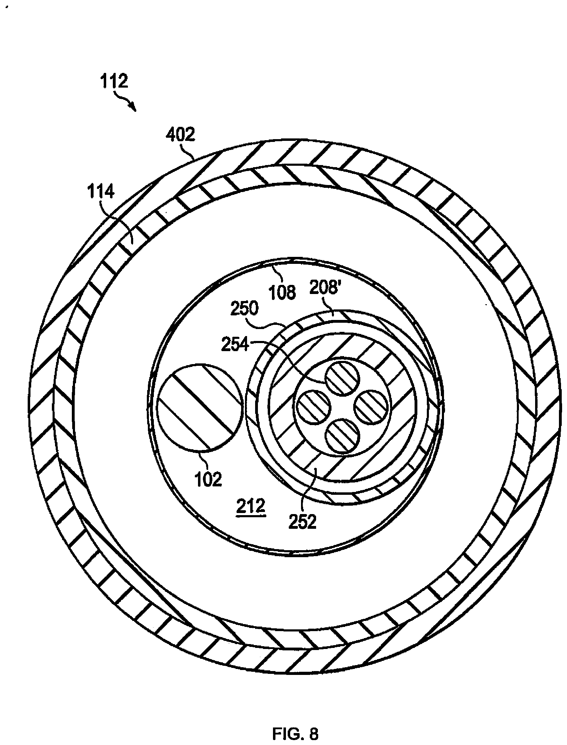

Fig. 4 A is a diagram showing an illustrative cross-section of the distal junction 112 of the balloon catheter 100 according to one embodiment of the present disclosure. According to certain illustrative examples, the distal junction 112 connects DEB assembly 110 to mid-shaft 114 at the distal end of the balloon. The distal junction 112 includes the inner member 102, the inner balloon sleeve 108, and space 212 through which connection media 208 runs. Distal junction 112 further includes a balloon distal leg 402. Fig. 8 illustrates similar features including the rotary drive shaft assembly 208, which will not be described in further detail herein. For further discussion of these and other alternative features of various catheters, please refer to U.S. Non-Provisional Application No. 14/096,982, entitled "HIGH PRESSURE THERAPEUTIC AND IMAGING CATHETER," filed on December 4, 2013, owned by the Assignee of the present invention.

As mentioned above, DEB assembly 110 can be used to treat various types of treatment areas, such as arterial occlusions or lesions. When DEB assembly 110 is appropriately positioned within a patient's vessel, balloon outer sleeve 119 is then inflated to put pressure on the treatment area. Balloon outer sleeve 119 is typically inflated with an inflation fluid. The inflation fluid is typically a saline fluid as such a fluid is harmless to the patient if it leaks into the artery. The inflation fluid may be pumped into the balloon through an inner lumen of proximal shaft 104 to a range of 15 to 20 ATM, or even greater depending on material properties of the balloon. As DEB assembly 110 is inflated, drug eluting coating 120 is forced into contact with the treatment area. Once applied, in certain illustrative embodiments, drug eluting coating 120 provides a sustained release of the coating medication, such as an antiproliferative drug, such as paclitaxel. If properly positioned, the drug eluting coating 120 provides a homogenous drug transfer to the entire vessel wall along with a rapid release of high concentrations of the drug into vessel tissue in contact with the coating. Utilizing the imaging elements provided with the present disclosure, contact between the drug eluting coating and the vessel wall can be confirmed before removal of the balloon catheter.

In addition, the imaging elements can be utilized during the initial balloon expansion procedure to determine accurate drug placement within the vessel, evaluate wall opposition of the balloon against the vessel, and post dilation vessel sizing. In one aspect, the imaging elements may be utilized prior to balloon delivery to evaluate the vessel to determine the precise location for deployment of the balloon and associated drug coating as well as co-registering the image from the delivery system with other imaging and diagnostic modalities, such as angiography, fluoroscopy and pressure sensing guidewires, to ensure accurate placement in the proper location within the vessel. In still a further aspect, when utilized following previous intravascular treatments, the delivery system imaging elements can evaluate the previous treatments effectiveness and need for further treatment by imaging such features as prior drug dispersion into the vessel or lesion, restenosis within or adjacent a prior treatment including stents and drug coatings, and an evaluation of the apposition of prior stents to the vessel walls.

According to certain illustrative examples, balloon outer sleeve 119 is a non- compliant balloon. A non-compliant balloon is one that is designed to inflate to a particular diameter and not stretch beyond that diameter. This prevents balloon outer sleeve 119 from expanding too much. This is important because excess expansion could damage a patient's blood vessel. Balloon outer sleeve 119 may also be designed to resist too much axial compression, which could allow the non-compliant balloon outer sleeve 119 to expand farther than desired. Additionally, outer sleeve 119 may be designed to resist too much axial stretching, which could prevent the balloon outer sleeve 120 from expanding to the desired diameter. Nevertheless, as will be detailed below in Figs. 9-14, drug eluting coating 120 is positioned around out sleeve 119 for delivery to a treatment area. DEB assembly 110 is inflated to apply drug eluting coating 120 to the treatment area, whereby drug eluting coating 120 remains after the balloon has been deflated.

As mentioned above, sensing device 116 can be used to image the interior of a patient's blood vessel. Various types of sensing devices may be used. One example of a sensing device 116 is an OCT device. In another form, the sensor can collect information for spectroscopy or photo acoustic imaging. Sensing device 116 may also be a forward looking device that scans forward into the vessel rather than outward from the axis towards the vessel walls.

Sensing device 116 may also be an IVUS device. There are two general types of IVUS devices that may be used. The first type of device is a solid state device, also known as a phased array. Solid-state IVUS devices carry a transducer complex that includes an array of ultrasound transducers distributed around the circumference of the device. The transducers are connected to a set of transducer controllers. The transducer controllers select individual transducers for transmitting an ultrasound pulse and for receiving the echo signal. By stepping through a sequence of transmit-receive pairs, the solid-state IVUS system can synthesize the effect of a mechanically scanned transducer element, but without moving parts. Because there is no rotating mechanical element, the transducer array can be placed in direct contact with the blood and vessel tissue with minimal risk of vessel trauma. Furthermore, the interface is simplified because there is no rotating element. The solid-state scanner can be wired directly to the imaging system with a simple electrical cable and a standard detachable electrical connector.

In the example of a transducer array as a sensing device, the connection medium running through the catheter shafts includes the electrical cables that communicate data between the transducer array and external processing systems. The number of wires and cables comprising the connection media may depend on the type of transducer array. For example, a 64 bit array may use more cables than a 32 bit array. Additionally, various multiplexing functions may be used to reduce the number of wires running through the catheter shafts.

The second general type of IVUS device is a rotational device *(e.g.,* sensing device 117). A typical rotational IVUS device includes a single ultrasound transducer element located at the tip of a flexible driveshaft. The transducer can be a traditional planar PZT type transducer or the transducer can be a focused transducer such as, for example, a piezoelectric micromachined ultrasound transducer ("PMUT") type device that permits Focused Acoustic Computed Tomography ("FACT"). In certain embodiments, the transducer is positioned distally of DEB assembly 110, while in another embodiment the transducer is positioned within inner sleeve 108 within DEB assembly 110. In yet other embodiments, the transducer is moveable along inner sleeve 108 such that DEB assembly 110 and the transducer are moveable relative to one another. Nevertheless, in either embodiment, the driveshaft spins inside a plastic sheath inserted into the vessel of interest. The transducer element is oriented such that the ultrasound beam propagates generally perpendicular to the axis of the device. The fluid-filled sheath protects the vessel tissue from the spinning transducer and driveshaft while permitting ultrasound signals to propagate from the transducer into the tissue and back. As the driveshaft rotates, the transducer is periodically excited with a high voltage pulse to emit a short burst of ultrasound. The same transducer then listens for the returning echoes reflected from various tissue structures, the balloon and the drug coating. The IVUS imaging system assembles a two dimensional display of the vessel cross-section from a sequence of pulse/acquisition cycles occurring during a single revolution of the transducer.

In the example of a rotational array (e.g., sensing/imaging device 116), the connection media running through the catheter shafts includes a driveshaft lumen that comprises the plastic sheath 250 surrounding a driveshaft 252 (Figs. 4 and 6) used to drive the rotational array. Additionally, the connection media include any electrical cables 254 that communicate data between the transducer array and external processing systems.

Figs. 9-11 illustrate the insertion of a DEB catheter 500 into a patient according to certain illustrative methods of the present disclosure. DEB catheter 500 includes a DEB assembly 502 and an imaging device 503, which are substantially similar to the DEB assembly 110 and sensing device 116, respectively, except for any differences noted herein. Inner sleeve 504 of the DEB catheter 500 is substantially similar to inner sleeve 108, except for any differences noted herein. As mentioned above in relation to inner sleeve 108, in some embodiments, inner sleeve 504 has high pressure capability greater than 20 ATM, which makes the DEB assembly 502 suitable for non-compliant post dilatation. For example, Figs. 9-13 illustrate the use of the DEB catheter 500 to access an intravascular treatment area (e.g., lesion 506), assess intravascular lesion 506, and treat intravascular lesion 506 using drug eluting coating 508, according to one embodiment of the present disclosure.

Fig. 9 illustrates DEB catheter 500 being advanced into a patient's vessel 510. Initially, a guide-wire 512 is fed into the vessel 510. In one aspect, a guidewire having a diameter of approximately 0.014 inches can be utilized. DEB catheter 500 can then be moved along the guide-wire 502 deeper into the patient's vessel 510. During insertion of DEB catheter 500 into the vessel 510, DEB assembly 502 is not inflated and maintains a low profile in an unexpanded condition. A distal end 514 of the DEB catheter 500 can be designed to facilitate entry and progress through the vessel 510. For example, the distal end 514 may be tapered.

As shown in Fig. 9, DEB catheter 500 is pushed into the vessel 510 until imaging device 503 and a distal junction 516 of DEB assembly 502 enters vessel 510. DEB catheter 500 is then pushed further into vessel 510 until a proximal junction 518 of DEB assembly 502 enters the vessel 510. Thereafter DEB catheter 500 is pushed further into vessel 510 with a proximal shaft 520 extending outside vessel 510 and outside the patient.

Fig. 10 illustrates DEB catheter 500 moving through the lesion 506 in the patient's vessel 510. Imaging device 503 can be used to detect and assess lesion 506. Lesion 506 includes a proximal end 525 and a distal end 530, as well as a length LI extending from the proximal end 525 to the distal end 530. As DEB catheter 500 traverses vessel 510, a healthcare professional can view the data obtained by imaging device 503 to assess the health of vessel 510. The imaging data can inform the doctor if there is some type of intravascular lesion or injury, such as, by way of non-limiting example, the intravascular lesion 506. The imaging data may also relay other vascular characteristics, such as, by way of non-limiting example, the path and/or tortuosity of the vessel 510, the regularity or irregularity of the walls within vessel 510, and various characteristics about the blood flow within the vessel 510.

Upon visualizing lesion 506, DEB catheter 500 is advanced further into the vessel 510 until DEB assembly 502 is aligned with lesion 506. Imaging device 503 can continue to image the vessel as the distal end 514 of DEB catheter 500 travels through lesion 506, thereby providing the healthcare professional with an accurate assessment of the location of DEB assembly 502. In particular, imaging device 503 is positioned a fixed distance D1 from DEB assembly 502, which allows a healthcare professional to advance and/or retract DEB catheter 500 the fixed distance to position DEB assembly 502 relative to whatever intravascular position imaging device 503 is imaging at a given time.

Imaging device 503 can also be used to facilitate placement of DEB assembly 502 adjacent to lesion 506. In the illustrated example, lesion 506 is an intravascular occlusion that requires reduction and coating as treatment. As shown in Figs. 10 and 11, as imaging device 503 travels through lesion 506, the image data relayed by imaging device 503 can inform the healthcare professional of various anatomic characteristics within vessel 510, such as, by way of non-limiting example, the length LI of lesion 506, the luminal contours of lesion 506 (e.g., the intraluminal diameter of the vessel 510 proximal, adjacent, and distal to lesion 506), and characteristics of the blood flow through lesion 506. Using this imaging data, the healthcare professional can advance DEB catheter 500 an appropriate distance forward to accurately position the unexpanded DEB assembly 502 and overlying drug eluting coating 508 within lesion 506. In certain embodiments, drug eluting coating 508 includes a length L2 extending from a proximal end 535 to a distal end 540. The healthcare professional can assess whether the length L2 of drug eluting coating 508 is appropriate to treat lesion 506, which has the length LI. In addition, the healthcare professional may verify that the diameter of drug eluting coating 508 is appropriate to treat lesion 506. If drug eluting coating 508 is comparatively too short, too long, too wide, or too slender to appropriately treat lesion 506, DEB catheter 500 may be removed and replaced with a catheter carrying a correctly-sized drug eluting coating 508, thereby avoiding the failure to the coating to completely cover lesion 506.

Fig. 11 illustrates the expansion of DEB assembly 502 and drug eluting coating 508 within lesion 506 in the patient's vessel 510. After the healthcare professional advances the DEB assembly 502 and drug eluting coating 508 (in an unexpanded condition) appropriately within lesion 506, the healthcare professional may inflate DEB assembly 502 to both relieve the occlusion caused by lesion 506 and apply drug eluting coating 508 to lesion 506, thus maintaining the new patency of the vessel 510 at the location of lesion 506. As mentioned above, this may be done by pumping an inflation fluid through an inner lumen of the proximal shaft 520 of DEB catheter 500. As DEB assembly 502 is inflated under a high pressure, typically in the range of 15-25 ATM, drug eluting coating 508 assumes an expanded condition and flattens lesion 506 against inner walls of the vessel 510, while at the same time adhering to lesion 506.

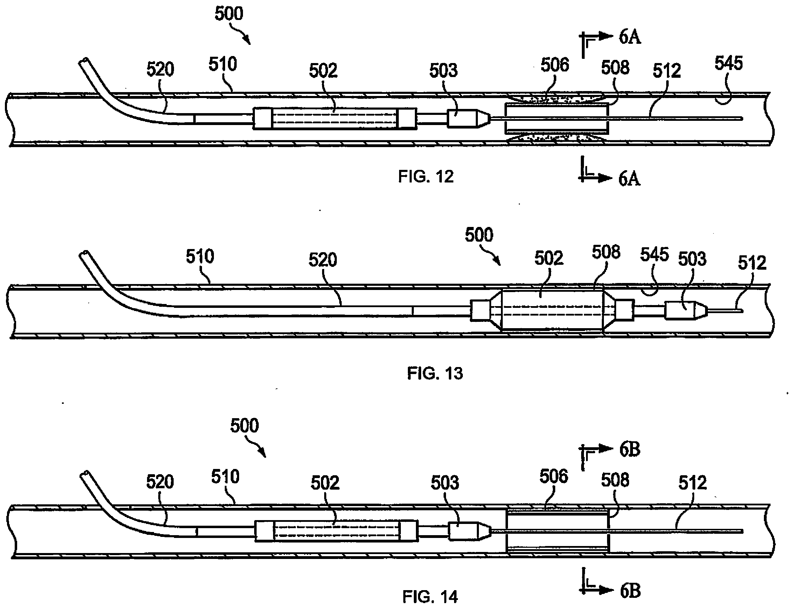

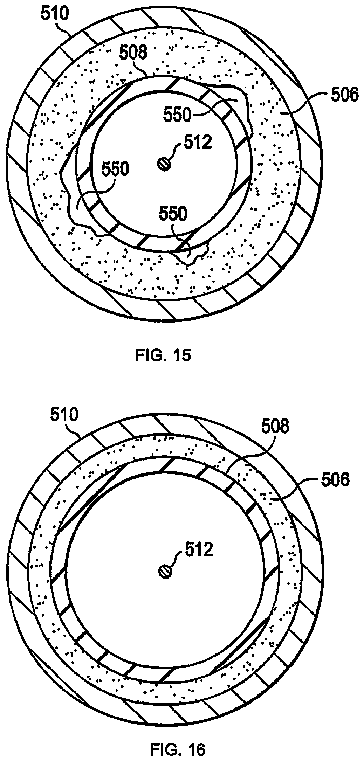

Fig. 12 illustrates the withdrawal of DEB assembly 502 from lesion 506 after initial application of drug eluting coating 508 on lesion 506. The healthcare professional may deflate DEB assembly 502 and retract DEB catheter 500 until imaging device 503 is positioned proximal to drug eluting coating 508. The healthcare professional can use imaging data received by imaging device 503, now positioned adjacent to lesion 506 and drug eluting coating 508, to assess the expansion and application of drug eluting coating 508. In particular, the imaging data allows the healthcare professional to assess the apposition of drug eluting coating 508 and lesion 506 within vessel 510. Occasionally, as shown in Fig. 12, the initial expansion of DEB assembly 502/drug eluting coating 508 is insufficient to result in complete apposition of drug eluting coating 508 and lesion 506, thus resulting in voids 550 as shown in Fig. 15. For example, in the pictured embodiment, drug eluting coating 508 has not completely coated or compressed lesion 506 against wall 545 of vessel 510. Instead, lesion 506 remains partially intact and capable of at least partially occluding flow through the vessel 510 or continuing its growth since it is not completely coated. Imaging device 503 can convey this information via imaging data to the healthcare professional.

Fig. 13 illustrates the reinsertion and re-expansion of DEB assembly 502 within lesion 506. After assessing the apposition of drug eluting coating 508 and lesion 506, if the healthcare professional desires to increase the expansion of the DEB assembly 502, eliminate voids 550, and/or further decrease the profile of lesion 506, the healthcare professional may re- advance DEB catheter 500 and re-position DEB assembly 502 within drug eluting coating 508 and lesion 506. As shown in Fig. 13, DEB assembly 502 may be re-inflated at a higher pressure to further expand drug eluting coating 508 against lesion 506, thereby improving the apposition and eliminating and/or reducing the presence of voids 550.

For example, if the initial inflation pressure was 17 ATM, the subsequent inflation pressure may be 20 ATM. In another example, if the initial inflation pressure was 20 ATM, the subsequent inflation pressure may be 25 ATM. Other changes in pressure between the initial and subsequent pressure are contemplated. In some embodiments, the subsequent pressure may be greater than the initial pressure by a predetermined percentage. For example, in one instance, the subsequent inflation pressure may be at least 25% greater than the initial inflation pressure. Other predetermined percentage increases are contemplated. In some embodiments, the healthcare provider may select the change or delta between the initial pressure and the subsequent pressure depending upon the desired degree of further expansion of the treatment device.

Fig. 14 illustrates the withdrawal of DEB assembly 502 from lesion 506 after the secondary application of drug eluting coating 508 to lesion 506. The healthcare professional may once again deflate DEB assembly 502 and retract DEB catheter 500 until imaging device 503 is positioned proximal to drug eluting coating 508. The healthcare professional can use imaging data received by imaging device 503 to assess the apposition between drug eluting coating 508 and lesion 506. If the imaging data indicates complete apposition (in addition to appropriate positioning, for example) as shown in Fig. 16 (voids 550 have been eliminated), then the healthcare professional may withdraw DEB catheter 500 from the vessel 510 (and the patient's body).

As described above with reference to Figs. 9-14, in certain other embodiments, the healthcare professional may inflate DEB assembly 502 at increasingly higher pressures in combination with imaging to verify the accurate positioning, repositioning, and real-time use of drug eluting coating 508.

Although not shown, in certain alternative methods, DEB catheter 500 may utilize a rotational imaging device *(e.g.,* sensor 117) as previously mentioned and illustrated in Fig. 2. With reference to Figs. 2 and 9-14, in such embodiments after imaging device 117 has imaged lesion 506, imaging device 117 may be held in a stationary position adjacent or within lesion 506. Since DEB assembly 502 and imaging device 117 are movable relative to one another in such embodiments, DEB assembly 502 may be moved in relation to imaging device 117 such that DEB assembly 502 is positioned over imaging device 117 (and within lesion 506 since imaging device 117 is positioned therein as well). Thereafter, DEB assembly 502 may be expanded as described herein. While DEB assembly 502 is inflated, imaging device 117 may then be moved through DEB assembly 502 to thereby image the apposition of drug eluting coating 508 and lesion 506. If the apposition is found to be incomplete, DEB assembly 502 may be further inflated at a higher pressure in efforts to minimize and/or eliminate any voids 550 that are present. The imaging and further inflation process may then be repeated as desired.

In yet other illustrative embodiments of the present disclosure, distance markers may be positioned along the shaft of the DEB catheter that protrudes outside the patient's body. During the treatment procedures described herein, the position of the distance markers may be viewed in relation to a fixed component *(e.g.,* access catheter, luer lock, etc.) to thereby move the DEB catheter the fixed distance described above to bring the DEB assembly to the previous position of the imaging device or vice versa. In other embodiments, the distance markers may be positioned on the DEB assembly itself, whereby the imaging device is used to view the distance markers so that the necessary movement of the DEB catheter is determined. In yet other embodiments, fluoroscopy may be used to image radiopaque marker bands along the exterior of the catheter.

The illustrative embodiments described herein may be utilized in a variety of applications, including, for example, the treatment of lesions and coronary artery stenosis. In addition, the illustrative DEB catheters could be used for renal denervation procedures. In such applications, a slow acting drug is utilized as the drug eluting coating that lasts 30-90 days and desensitizes the nerves. This gives the patient a trial procedure to determine if the technique relieves hypertension without permanently damaging the nerves. In an alternative application, the slow acting drug eluting coating may permanently damage the nerves to thereby accomplish denervation. These and other applications of the present invention will be apparent to those ordinarily skilled in the art having the benefit of this disclosure.

Although illustrative embodiments have been shown and described, a wide range of modification, change, and substitution is contemplated in the foregoing disclosure and in some instances, some features of the present disclosure may be employed without a corresponding use of the other features. It is understood that such variations may be made in the foregoing without departing from the scope of the present disclosure. Accordingly, it is appropriate that the appended claims be construed broadly and in a manner consistent with the scope of the present disclosure.

## Claims

1. An integrated therapeutic and imaging catheter (100), comprising:
an inner member (102) defining a guidewire lumen (103);
a balloon assembly (110) positioned over the inner member;
a drug eluting coating positioned on the balloon assembly; and
an imaging device (117) positioned over the inner member,
**characterised in that** the imaging device (117) and the balloon assembly are movable longitudinally relative to one another.

2. The catheter as defined in claim 1, wherein the imaging device (117) comprises at least one of an intravascular ultrasound transducer and an optical coherence tomography device.

3. The catheter as defined in claim 1, wherein the imaging device and balloon assembly are positionable separately from one another at a fixed distance.

4. The catheter as defined in claim 1, further comprising distance markers positioned on at least one of the catheter extending outside a body of a patient or the balloon assembly.

5. The catheter as defined in claim 1, wherein the drug eluting coating includes paclitaxel.

6. The catheter as defined in claim 1, wherein the balloon assembly is non-compliant.

7. The catheter as defined in claim 1, wherein the imaging device (117) is a rotational imaging device.

8. The catheter as defined in claim 7, wherein the rotational imaging device (117) is rotatable inside a mid-shaft (114) housing the inner member (102).

9. The catheter as defined in claim 1, wherein the inner member (102) runs through the balloon assembly and the mid-shaft (114).

## Patentansprüche

1. Ein integrierter Behandlungs- und Bildgebungskatheter (100), der Folgendes umfasst:
ein inneres Element (102), das ein Führungsdrahtlumen (103) bildet;
eine Ballonanordnung (110)über dem inneren Element;
eine ein Medikament freisetzende Beschichtung auf der Ballonanordnung; und
ein Bildgebungsgerät (117)über dem inneren Element;
der sich dadurch auszeichnet, dass
das Bildgebungsgerät (117) und die Ballonanordnung relativ zueinander in Längsrichtung bewegt werden können.

2. Der Katheter gemäß Anspruch 1, wobei das Bildgebungsgerät (117) mindestens einen intravaskulären Ultraschallwandler oder ein optisches Kohärenztomografiegerät umfasst.

3. Der Katheter gemäß Anspruch 1, wobei das Bildgebungsgerät und die Ballonanordnung separat von einander in einem festen Abstand positioniert werden können.

4. Der Katheter gemäß Anspruch 1, wobei dieser zudem Abstandsgeber mindestens entweder auf dem Katheter, der aus dem Körper eines Patienten hervorsteht, oder auf der Ballonanordnung umfasst.

5. Der Katheter gemäß Anspruch 1, wobei die ein Medikament freisetzende Beschichtung Paclitaxel enthält.

6. Der Katheter gemäß Anspruch 1, wobei die Ballonanordnung nicht konform ist.

7. Der Katheter gemäß Anspruch 1, wobei es sich beim Bildgebungsgerät (117) um ein sich drehendes Bildgebungsgerät handelt.

8. Der Katheter gemäß Anspruch 7, wobei das sich drehende Bildgebungsgerät (117) in einem Gehäuse des mittleren Schafts (114) im inneren Element (102) dreht.

9. Der Katheter gemäß Anspruch 1, wobei das innere Element (102) durch die Ballonanordnung und den mittleren Schaft (114) verläuft.

## Revendications

1. Cathéter thérapeutique intégré et d'imagerie (100), comprenant :
un élément interne (102) définissant une lumière pour fil de guidage (103) ;
un ensemble à ballonnet (110) positionné sur l'élément interne ;
un revêtement imprégné de médicament positionné sur l'ensemble à ballonnet ; et
un dispositif d'imagerie (117) positionné sur l'élément interne, **caractérisé en ce que** le dispositif d'imagerie (117) et l'ensemble à ballonnet se déplacent de façon longitudinale l'un par rapport à l'autre.

2. Cathéter selon la revendication 1, dans lequel le dispositif d'imagerie (117) comprend au moins un transducteur ultrasonore intravasculaire et un dispositif de tomographie de cohérence optique.

3. Cathéter selon la revendication 1, dans lequel le dispositif d'imagerie et l'ensemble à ballonnet sont positionnables séparément l'un de l'autre à une distance fixe.

4. Cathéter selon la revendication 1, comprenant en outre des marqueurs de distance positionnés sur au moins un cathéter s'étendant à l'extérieur d'un corps d'un patient ou l'ensemble à ballonnet.

5. Cathéter selon la revendication 1, dans lequel le revêtement imprégné de médicament comprend du paclitaxel.

6. Cathéter selon la revendication 1, dans lequel l'ensemble à ballonnet est non-conforme.

7. Cathéter selon la revendication 1, dans lequel le dispositif d'imagerie (117) est un dispositif d'imagerie rotatif.

8. Cathéter selon la revendication 7, dans lequel le dispositif d'imagerie rotatif (117) tourne à l'intérieur d'un arbre médian (114) logeant l'élément interne (102).

9. Cathéter selon la revendication 1, dans lequel l'élément interne (102) passe par l'ensemble à ballonnet et l'arbre médian (114).
